# EUROPEAN PATENT APPLICATION

(11) **EP 4 474 476 A1**
(43) Date of publication of application: **11.12.2024**
(21) Application number: 23749871.2
(22) Date of filing: 03.02.2023
(51) Int. Cl.: C12N 5/0783, A61K 35/17, A61K 39/395, A61P 35/00, C07K 19/00, C12N 5/10, C12N 15/62

(54) **T CELL PRODUCTION METHOD**

(30) Priority: 04.02.2022 JP 2022016271
(71) Applicant: Kyoto University, Kyoto-shi, Kyoto 606-8501 (JP); Takeda Pharmaceutical Company Limited, Osaka-shi, Osaka 541-0045 (JP)
(72) Inventor: KANEKO, Shin, Kyoto-shi, Kyoto 606-8501 (JP); IRIGUCHI, Shoichi, Kyoto-shi, Kyoto 606-8501 (JP); MIYAKE, Yasuyuki, Kyoto-shi, Kyoto 606-8501 (JP); SHINOHARA, Tokuyuki, Fujisawa-shi, Kanagawa 251-0012 (JP); SEKIYA, Keiko, Fujisawa-shi, Kanagawa 251-0012 (JP)
(74) Representative: Jones, Nicholas Andrew
(86) International application number: PCT/JP2023/003623
(87) International publication number: WO 2023/149555

(57) **Abstract**

Disclosed is a method for producing T cells, the method comprising: (1) culturing three-dimensional cell aggregate(s) comprising cells that can differentiate into T cells, and stromal cells that express Notch ligand(s) derived from pluripotent stem cells. Also disclosed are T cells obtained by the method, and a medicine comprising the T cells.

## Description

### Technical Field

The present invention relates to a method for producing T cells, T cells obtained by the method, a medicine comprising the T cells, and the like.

### Background Art

In recent years, research and development of various cell therapies using T cells (e.g., CAR T cell therapy and regulatory T cell (Treg cell) therapy) has been actively promoted around the world. When inducing differentiation of iPS cells into T cells, it is known that conventional 2D culture methods mainly induce CD8 single-positive (SP) T cells, and that it is difficult to induce other T cells (e.g., CD4 SP T cells).

On the other hand, PTL 1 and NPL 1 disclose a method for preparing a composition of T cells from stem cells or progenitor cells by culturing three-dimensional cell aggregate(s) comprising a selected population of stromal cells expressing Notch ligand(s) and a selected population of stem cells or progenitor cells, which is called the artificial thymic organoid (ATO) method.

In addition, PTL 2 discloses a genetically engineered mammalian cell comprising a transgene encoding a lineage commitment factor that promotes differentiation into CD4⁺ Treg.

### Citation List

### Patent Literature

PTL 1: WO2017/075389
PTL 2: WO2021/092581

### Non-patent Literature

NPL 1: Nat. Methods, 14, 521-530 (2017)

### Summary of Invention

### Technical Problem

An object of the present invention is to provide a method for producing T cells with high safety in production.

### Solution to Problem

As described above, the ATO method is known as a method for inducing differentiation of iPS cells or the like into various T cells (CD4 SP cells, CD8 SP cells, γδ T cells, regulatory T cells, and the like). In the ATO method, mouse stromal cells are often used as feeder cells; however, considering production hurdles from the standpoint of good manufacturing protocol (GMP), it was preferable to use xeno-free methods.

However, no xeno-free methods have been reported so far. For example, when primary human fibroblasts are used as feeder cells, disadvantages include limited proliferation ability and/or large donor-dependent variation or lot-to-lot variation. In contrast, when using human fibroblast lines as feeder cells, there are no disadvantages as in when using primary human fibroblasts; however, the disadvantage is that since they are cancer cells, there is a possibility that there may be an increased risk in clinical application.

As a result of extensive studies to achieve the above object, the present inventors found that it is possible to provide a method for producing T cells with high safety in production by producing stromal cells (particularly fibroblasts) derived from human iPS cells and using them as feeder cells in the ATO method.

The present invention has been completed upon further examination based on this finding. The present invention provides a method for producing T cells, T cells, medicines, and the like described below.
[1] A method for producing T cells, the method comprising:
   (1) culturing three-dimensional cell aggregate(s) comprising cells that can differentiate into T cells, and stromal cells that express Notch ligand(s) derived from pluripotent stem cells.
   [2] The method according to [1], wherein the cells that can differentiate into T cells are CD34⁺ cells or mesodermal progenitor cells.
   [3] The method according to [1], wherein the cells that can differentiate into T cells are hematopoietic stem cells, hemogenic endothelial cells, progenitor T cells, or mesodermal progenitor cells.
   [4] The method according to [1], wherein the cells that can differentiate into T cells are hemogenic endothelial cells.
   [5] The method according to any one of [1] to [4], wherein the cells that can differentiate into T cells are cells derived from pluripotent stem cells.
   [6] The method according to any one of [1] to [5], wherein the cells that can differentiate into T cells are cells derived from iPS cells.
   [6a] The method according to any one of [1] to [6], wherein the cells that can differentiate into T cells are cells derived from human iPS cells.
   [7] The method according to any one of [1] to [6a], wherein the stromal cells are fibroblasts.
   [8] The method according to any one of [1] to [7], wherein the pluripotent stem cells are iPS cells.
   [8a] The method according to any one of [1] to [8], wherein the pluripotent stem cells are human iPS cells.
   [9] The method according to any one of [1] to [8a], wherein the Notch ligand(s) is at least one member selected from the group consisting of DLL4, DLL1, JAG1, and JAG2.
   [10] The method according to any one of [1] to [9], further comprising, before step (1):
      (2) inducing differentiation of pluripotent stem cells into stromal cells.
   [11] The method according to any one of [6] to [10], further comprising, before step (1):
      (3) inducing differentiation of iPS cells into cells that can differentiate into T cells.
   [12] The method according to any one of [1] to [11], further comprising, before step (1):
      (4) introducing a nucleic acid encoding an exogenous T-cell receptor and/or a chimeric antigen receptor into cells that can differentiate into T cells.
   [13] The method according to [11] or [12], further comprising, before step (3):
      (5) introducing a nucleic acid encoding an exogenous T-cell receptor and/or a chimeric antigen receptor into iPS cells.
   [14] The method according to any one of [1] to [13], which is a method for producing T cells comprising at least one selected from the group consisting of CD4⁺ T cells, CD8⁺ T cells, γδ T cells, and regulatory T cells.
   [14a] The method according to any one of [1] to [13], which is a method for producing T cells comprising at least one selected from the group consisting of CD4⁺ T cells, CD8⁺ T cells, and γδ T cells.
   [14b] A cell population derived from pluripotent stem cells, wherein the ratio of CD4⁺ T cells to CD8⁺ T cells in αβT cells contained in the cell population is 0.6 or more.
   [15] T cells obtained by the method according to any one of [1] to [14a].
   [16] A medicine comprising the T cells according to [15] .
   [17] A method for producing T cells expressing an exogenous T-cell receptor and/or a chimeric antigen receptor, comprising:
      (6) introducing a nucleic acid encoding an exogenous T-cell receptor and/or a chimeric antigen receptor into T cells obtained by the method according to any one of [1] to [14a].
   [18] The medicine according to [16], which is a preventive and/or therapeutic agent for a T cell-related disease.
   [19] The medicine according to [18], wherein the T cell-related disease is a tumor, an infection, or a blood disease.
   [19a] The medicine according to [18], wherein the T cell-related disease is an abnormally enhanced immune response.
   [20] A method for preventing and/or treating a T cell-related disease, comprising administering the T cells according to [15] to a subject in need thereof.
   [21] The method according to [20], wherein the T cell-related disease is a tumor, an infection, or a blood disease.
   [21a] The method according to [20], wherein the T cell-related disease is an abnormally enhanced immune response.
   [22] T cells according to [15] for use in the prevention and/or treatment of a T cell-related disease.
   [23] The T cells according to [22], wherein the T cell-related disease is a tumor, an infection, or a blood disease.
   [23a] The T cells according to [22], wherein the T cell-related disease is an abnormally enhanced immune response.
   [24] Use of the T cells according to [15] in the production of a medicine for use in the prevention and/or treatment of a T cell-related disease.
   [25] The use according to [24], wherein the T cell-related disease is a tumor, an infection, or a blood disease.
   [25a] The use according to [24], wherein the T cell-related disease is an abnormally enhanced immune response.

### Advantageous Effects of Invention

The present invention provides a method for producing T cells with high safety in production. Further, in the production method of the present invention, stromal cells derived from pluripotent stem cells are used as feeder cells, so that the lot-to-lot variation of feeder cells used is small, and T cells can be stably produced. According to an embodiment of the present invention, T cells with excellent anticancer activity can be produced. Further, according to an embodiment of the present invention, T cells with excellent cellular kinetics can be produced.

### Brief Description of Drawings

Fig. 1 shows the results of flow cytometry analysis of the expression of fibroblast markers CD90 and DLL4 in iPS cell-derived fibroblasts and MS5 introduced with human DLL4 gene.
Fig. 2 shows the results of flow cytometry analysis of CD3/TCRαβ, CD4/CD8α, CD8α/CD8β, and CAR/IL15Rα after induction of differentiation of iPS cells into T cells. From top to bottom, the results are shown for FfI01s04+MS5/DLL4, FfI01s04+iFibro/DLL4, QHJI01s04/G9D2+MS5/DLL4, and QHJI01s04/G9D2+iFibro/DLL4.
Fig. 3 shows photographs illustrating the in vivo drug efficacy of iPS cell-derived CD19CAR T cells. From top to bottom, the results are shown for 1, 2, 3, 4 and 5 weeks after administration.
Fig. 4 is a graph showing the in vivo cellular kinetics of iPS cell-derived CD19CAR T cells. The number of cells after 1, 2, 3, 4, and 5 weeks after administration of cells is shown, and the values in the graph are mean ± SD.
Fig. 5 shows the results of examining the expression levels of proteins expressed in Tregs, which were differentiated from iPS cells by using iPS cell-derived fibroblasts. The dot plot in each figure shows a CD3⁺CD4⁺CD8⁻population. Further, the numbers in each graph indicate the proportion of Treg (CD3⁺/CD4⁺/CD8⁻/CD25⁺/FOXP3⁺) in each cell population.

### Description of Embodiments

Embodiments of the present invention are described in detail below.

The term "comprise(s)" or "comprising" means that although elements following these terms are included, the inclusion is not limited to the elements. Therefore, these terms suggest inclusion of elements following them, but do not suggest exclusion of any other elements. The term "consist(s) of" or "consisting of" means that any elements following these terms are included, and that the inclusion is limited to the elements. Therefore, the term "consist(s) of" or "consisting of" indicates that the listed elements are required or essential, and that there are substantially no other elements. The term "consist(s) essentially of" or "consisting essentially of" means that any elements following these terms are included, and that there is a limitation to other elements that do not affect the activity or action specified in the present disclosure for the above elements. Therefore, the term "consist(s) essentially of" or "consisting essentially of" indicates that the listed elements are required or essential, while other elements are optional, and may be or may not be present depending on whether they affect the activity or action of the listed elements.

In the present specification, the term "culture" refers to maintenance or/and proliferation of cells in an in-vitro environment. The term "culturing" refers to maintaining or/and proliferating cells outside tissue or outside the body (e.g., in a cell culture dish or flask).

In the present specification, the term "positive (+)" means that a protein or gene is expressed in detectable amounts by methods known in the art. In the case of a protein that is expressed intracellularly and does not appear on the cell surface (e.g., a transcription factor or a subunit thereof), a reporter protein is expressed together with the protein, and the reporter protein is detected, whereby the target protein can be detected. Gene detection can be performed by using nucleic acid amplification and/or nucleic acid detection methods, such as RT-PCR, microarray, biochip, and RNAseq.

In the present specification, the term "negative (-)" means that the expression level of a protein or gene is below the lower limit of detection by all or any of the known methods described above. The lower limit of detection of protein or gene expression may vary depending on the method.

In the present specification, the term "marker" refers to a protein or its gene that is specifically expressed on the cell surface, in the cytoplasm, or in the nucleus of a given cell type. The marker is preferably a "cell surface marker." The "cell surface marker" refers to a protein expressed on the cell surface that can be labeled (stained) with fluorescent substances and that facilitates the detection, condensation, isolation, or the like of cells expressing the cell surface marker. The cell surface marker refers to a gene that is expressed (positive marker) or not expressed (negative marker) specifically in a given cell type, and specifically a substance that is produced (positive marker) or not produced (negative marker) as mRNA by transcription of the gene in the genome or as a protein by translation of the mRNA.

Such cell surface markers can be detected by immunological assays using antibodies specific for the cell surface markers, such as ELISA, immunostaining, and flow cytometry.

In the present specification, the term "expression" is defined as the transcription and/or translation of a specific nucleotide sequence driven by an intracellular promoter.

In the present specification, "pluripotent stem cells" refer to embryonic stem cells (ES cells) and cells with similar pluripotency, i.e., cells with the potential to differentiate into various tissues (endoderm, mesoderm, and ectoderm) of a living body. Examples of cells with the same pluripotency as that of ES cells include induced pluripotent stem cells (also referred to as "iPS cells" in the present specification).

In the present specification, "hematopoietic stem cells (HSC)" are multipotent stem cells that can differentiate into blood cells. Hematopoietic stem cells are mainly present in the bone marrow in a human living body, and differentiate into white blood cells (neutrophils, eosinophils, basophils, lymphocytes, monocytes, and macrophages), red blood cells, platelets, mast cells, dendritic cells, and the like. In the present specification, hematopoietic stem cells (HSC) can be positive to CD34 and negative to CD7 (CD34⁺/CD7⁻). In the present specification, "/" used in the phrase "CD34⁺/CD7⁻" etc. means "and."

In the present specification, "hemogenic endothelial cells (HEC)" are cells that express CD34 but do not express CD43, CD184, and CD73 (CD34⁺/CD43⁻/CD184⁻/CD73⁻) (CD34⁺/CD43⁻/CD184⁻/CD73⁻ cells do not express CD7, and are thus also referred to as "CD34⁺/CD7⁻/CD43⁻/CD184⁻/CD73⁻ cells") .

In the present specification, "progenitor T cells (proT)" refer to hematopoietic cells produced in the process of differentiation from hematopoietic stem cells into CD3-positive T cells in a human living body, and are positive to CD34 and CD7 (CD34⁺/CD7⁺ cells). Further, in the present specification, proT can be negative to CD43, CDla and/or CD116.

In the present specification, "mesodermal progenitor cells" refer to, for example, cells that express at least one marker gene selected from the group consisting of T (synonymous with Brachyury), KDR, FOXF1, FLK1, BMP4, MOX1, and SDF1. Mesodermal progenitor cells are not distinguished from mesodermal cells. Cells with weak expression of the above marker genes may be referred to as "mesodermal progenitor cells."

In the present specification, "CD4CD8 double-positive T cells" refer to, among T cells, those with surface antigens CD4 and CD8 that are both positive (CD8⁺/CD4⁺). Since T cells can be identified by surface antigens CD3 and CD45 being positive, CD4CD8 double-positive T cells can be identified as cells that are positive to CD4, CD8, CD3, and CD45 (CD8⁺/CD4⁺/CD3⁺/CD45⁺ cells) .

In the present specification, "CD4CD8 double-negative T cells" refer to, among T cells, those with surface antigens CD4 and CD8 that are both negative (CD8⁻/CD4⁻). Since T cells can be identified by surface antigens CD3 and CD45 being positive, CD4CD8 double-negative T cells can be identified as cells that are negative to CD4 and CD8, and positive to CD3 and CD45 (CD8-/CD4-/CD3⁺/CD45⁺ cells).

In the present invention, hematopoietic stem cells, hemogenic endothelial cells, progenitor T cells, mesodermal progenitor cells, CD4CD8 double-positive T cells, and CD4CD8 double-negative T cells may be cells isolated from biological tissues, such as bone marrow, umbilical cord blood, and blood, or may be cells derived from pluripotent stem cells, such as ES cells and iPS cells.

In the present specification, "CD4⁺ T cells" refer to, among T cells, those with surface antigens CD4 and CD8 that are respectively positive and negative (CD4⁺/CD8⁻). Since T cells can be identified by surface antigens CD3 and CD45 being positive, CD4⁺ T cells can be identified as cells that are negative to CD8 and positive to CD4, CD3, and CD45 (CD4⁺/CD8⁻/CD3⁺/CD45⁺ cells). Examples of CD4⁺ T cells include helper T cells.

In the present specification, "CD8⁺ T cells" refer to, among T cells, those with surface antigens CD8 and CD4 that are respectively positive and negative (CD8⁺/CD4⁻). Since T cells can be identified by surface antigens CD3 and CD45 being positive, CD8⁺ T cells can be identified as cells that are negative to CD4 and positive to CD8, CD3, and CD45 (CD4-/CD8⁺/CD3⁺/CD45⁺ cells) . Examples of CD8⁺ T cells include cytotoxic T cells.

In the present specification, "γδ T cells" refer to cells that express CD3 and also express a T cell receptor (TCR) composed of a TCR γ chain (γTCR) and a TCR δ chain (δTCR).

In the present specification, "αβ T cells" refers to cells that express CD3 and also express a T cell receptor (TCR) composed of a TCR α chain (αTCR) and a TCR β chain (βTCR).

In the present specification, "regulatory T cells" refer to T cells that have the ability to inhibit the activation of effector T cells when stimulated via T cell receptors and that are responsible for the suppression of immune responses (immune tolerance). Regulatory T cells are generally CD25⁺/FOXP3⁺ cells, and CD4⁺ or CD8⁺ cells are present therein. In the present invention, the regulatory T cells may be CD4⁺ cells (i.e., CD4⁺/CD25⁺/FOXP3⁺) or CD8⁺ cells (i.e., CD8⁺/CD25⁺/FOXP3⁺), more preferably CD4⁺ cells, and even more preferably CD4⁺/CD8⁻/CD25⁺/FOXP3⁺ cells. Further, the transcription factor FOXP3 is known as a master regulator of CD4⁺/CD25⁺ regulatory T cells.

In the present specification, the "cell population" refers to two or more cells of the same or different types. The "cell population" also refers to a mass of cells of the same or different types.

From the viewpoint of therapeutic application, the various cells used in the present invention are preferably GMP-compliant cells.

Examples of pluripotent stem cells include embryonic stem cells (ES cells), induced pluripotent stem cells (iPS cells), embryonal carcinoma cells (EC cells), embryonic germ stem cells (EG cells), and Muse cells; and preferably iPS cells (more preferably human iPS cells). When the pluripotent stem cells are ES cells or any cells derived from the human embryo, the cells may be produced by destroying the embryo or without destroying the embryo, and preferably cells produced without destroying the embryo.

Regarding "ES cells," various mouse ES cell strains established by Ingenious Targeting Laboratory, RIKEN, etc. can be used as mouse ES cells, and various human ES cell strains established by the University of Wisconsin, NIH, RIKEN, Kyoto University, National Center for Child Health and Development, Cellartis, etc. can be used as human ES cells. Usable examples of human ES cell strains include CHB-1 to CHB-12 strains, RUES1 strain, RUES2 strain, HUES1 to HUES28 strains, etc. furnished by ESI Bio; H1 strain, H9 strain, etc. furnished by WiCell Research; and KhES-1 strain, KhES-2 strain, KhES-3 strain, KhES-4 strain, KhES-5 strain, SSES1 strain, SSES2 strain, SSES3 strain, etc. furnished by RIKEN.

"Induced pluripotent stem cells" refer to cells obtained by reprogramming mammalian somatic cells or undifferentiated stem cells by introduction with specific factors (nuclear reprogramming factors). Currently, there are many different types of induced pluripotent stem cells. Usable examples include iPS cells established by Yamanaka et al. by introducing four factors, Oct3/4, Sox2, Klf4, and c-Myc, into mouse fibroblasts (Takahashi K., Yamanaka S., Cell, (2006) 126: 663-676), as well as human cell-derived iPS cells established by introducing the same four factors into human fibroblasts (Takahashi K., Yamanaka S., et al. Cell, (2007) 131: 861-872), Nanog-iPS cells established by introducing the above four factors, followed by screening using the expression of Nanog as an index (Okita, K., Ichisaka, T., and Yamanaka, S. (2007). Nature 448, 313-317), iPS cells produced by a method that does not include c-Myc (Nakagawa M., Yamanaka S., et al. Nature Biotechnology, (2008) 26, 101-106), and iPS cells established by introducing six factors by a virus-free method (Okita K. et al. Nat. Methods 2011 May; 8(5): 409-12, Okita K. et al. Stem Cells. 31(3): 458-66). Other usable examples include induced pluripotent stem cells produced by Thomson et al. and established by introducing four factors, OCT3/4, SOX2, NANOG, and LIN28 (Yu J., Thomson J.A. et al., Science (2007) 318: 1917-1920), induced pluripotent stem cells produced by Daley et al. (Park I.H., Daley G.Q. et al., Nature (2007) 451: 141-146), induced pluripotent stem cells produced by Sakurada et al. (JP2008-307007A), and the like.

Other usable examples are any of the induced pluripotent stem cells known in the art and disclosed in all of the published articles (e.g., Shi Y., Ding S., et al., Cell Stem Cell, (2008) Vol. 3, Issue 5, 568-574; Kim J.B., Scholer H.R., et al., Nature, (2008) 454, 646-650; Huangfu D., Melton, D.A., et al., Nature Biotechnology, (2008) 26, No. 7, 795-797), or patents (e.g., JP2008-307007A, JP2008-283972A, US2008/2336610, US2009/047263, WO2007/069666, WO2008/118220, WO2008/124133, WO2008/151058, WO2009/006930, WO2009/006997, and WO2009/007852).

As induced pluripotent stem cell strains, various iPS cell strains established by NIH, RIKEN, Kyoto University, etc. can be used. Examples of human iPS cell strains include HiPS-RIKEN-1A strain, HiPS-RIKEN-2A strain, HiPS-RIKEN-12A strain, and Nips-B2 strain (RIKEN); Ff-I01s04 strain, QHJI strain, RWMH strain, DRXT strain, RJWI strain, YZWJ strain, ILCL strain, GLKV strain, 253G1 strain, 201B7 strain, 409B2 strain, 454E2 strain, 606A1 strain, 610B1 strain, and 648A1 strain (Kyoto University); and the like.

The "nucleic acid" may be any molecule obtained by polymerizing a nucleotide and a molecule having the same function as the nucleotide. Examples include RNA that is a polymer of ribonucleotide, DNA that is a polymer of deoxyribonucleotide, a polymer that is a mixture of ribonucleotide and deoxyribonucleotide, and a nucleotide polymer containing a nucleotide analog. The nucleic acid may also be a nucleotide polymer containing a nucleic acid derivative. The nucleic acid may be a single-stranded nucleic acid or a double-stranded nucleic acid. Double-stranded nucleic acids include a double-stranded nucleic acid in which one strand hybridizes to the other strand under stringent conditions.

The nucleotide analog may be any molecule as long as it is a molecule obtained by modifying ribonucleotide, deoxyribonucleotide, RNA, or DNA, for improvement of nuclease resistance, stabilization, increase in affinity with complementary strand nucleic acids, enhancement of cell permeability, or visualization, compared with RNA or DNA. The nucleotide analog may be a naturally occurring molecule or a non-natural molecule. Examples include sugar-modified nucleotide analogs (e.g., 2'-O-methylribose-substituted nucleotide analog, 2'-O-propylribose-substituted nucleotide analog, 2'-methoxyethoxyribose-substituted nucleotide analog, 2'-O-methoxyethylribose-substituted nucleotide analog, 2'-O-[2-(guanidium)ethyl]ribose-substituted nucleotide analog, 2'-fluororibose-substituted nucleotide analog, bridged nucleic acid (BNA), locked nucleic acid (LNA), ethylene-bridged nucleic acid (ENA), peptide nucleic acid (PNA), oxy-peptide nucleic acid (OPNA), and peptide ribonucleic acid (PRNA)), phosphodiester bond-modified nucleotide analogs (e.g., phosphorothioate bond-substituted nucleotide analog and N3'-P5' phosphoramidate bond-substituted nucleotide analog), and the like.

The nucleic acid derivative may be any molecule as long as it is a molecule obtained by adding another chemical substance to a nucleic acid, for improvement of nuclease resistance, stabilization, increase in affinity with complementary strand nucleic acids, enhancement of cell permeability, or visualization, compared with the nucleic acid. Specific examples include 5'-polyamine-adduct derivatives, cholesterol-adduct derivatives, steroid-adduct derivatives, bile acid-adduct derivatives, vitamin-adduct derivatives, Cy5-adduct derivatives, Cy3-adduct derivatives, 6-FAM-adduct derivatives, biotin-adduct derivatives, and the like.

The method for producing T cells of the present invention (also referred to simply as "the production method of the present invention" in the present specification) characteristically comprises the following step.

Step (1) is a step of culturing three-dimensional cell aggregate(s) containing cells that can differentiate into T cells, and stromal cells that express Notch ligand(s) derived from pluripotent stem cells.

The use of the production method comprising step (1) makes it possible to induce differentiation into T cells with high efficiency. The culture of the three-dimensional cell aggregate(s) in step (1) can be carried out using cells that can differentiate into T cells, and stromal cells that express Notch ligand(s) derived from pluripotent stem cells, with reference to the description in WO2017/075389 etc.

The cells that can differentiate into T cells used in step (1) are not particularly limited as long as they are cells that can differentiate into T cells. Examples include CD34⁺ cells, mesodermal progenitor cells, CD4CD8 double-negative T cells, CD4CD8 double-positive T cells, and the like; preferably CD34⁺ cells or mesodermal progenitor cells; more preferably hemogenic endothelial cells, hematopoietic stem cells, progenitor T cells, or mesodermal progenitor cells; and particularly preferably hemogenic endothelial cells. The CD34⁺ cells are cells that express CD34 (CD34⁺), and particularly cells that express CD34 and do not express CD7 (CD34⁺/CD7⁻). Examples of the CD34⁺ cells include hemogenic endothelial cells and hematopoietic stem cells.

The cells that can differentiate into T cells used in step (1) are more preferably cells differentiated from pluripotent stem cells (particularly iPS cells (especially human iPS cells)). Among these, the cells that can differentiate into T cells used in step (1) are desirably cells differentiated from pluripotent stem cells (particularly iPS cells (especially human iPS cells)) into CD34⁺ cells (particularly hemogenic endothelial cells).

The differentiation of the pluripotent stem cells into CD34⁺ cells can be induced according to a known method. When the pluripotent stem cells are iPS cells, the differentiation of hematopoietic progenitor cells can be induced, for example, by the methods disclosed in WO2017/221975, WO2018/135646, and Cell Reports 2 (2012) 1722-1735, thereby producing CD34⁺ cells. When hemogenic endothelial cells are subjected to step (1), step (1) can be performed on CD34⁺ cells containing hemogenic endothelial cells (i.e., not separating hemogenic endothelial cells before performing step (1)), or step (1) can also be performed on hemogenic endothelial cells separated according to a known method (e.g., flow cytometry or a magnetic cell separation method).

The stromal cells used are cells that express Notch ligand(s) and are derived from pluripotent stem cells. Stromal cells are a general term for connective tissue cells that support parenchymal cells, and representative examples include fibroblasts, blood cells, endothelial cells, smooth muscle cells, epithelial cells, and tissue stem cells. The stromal cells can be used singly or in combination of two or more. In addition, it is preferable that a nucleic acid for expressing Notch ligand(s) is introduced into the stromal cells. The stromal cells that express Notch ligand(s) can be produced by introducing the above nucleic acid into stromal cells. Alternatively, the stromal cells can also be produced by introducing the above nucleic acid into pluripotent stem cells (particularly iPS cells (especially human iPS cells)), and then differentiating the pluripotent stem cells into stromal cells.

The stromal cells used in the production method of the present invention are stromal cells differentiated from pluripotent stem cells (particularly iPS cells (especially human iPS cells)). In particular, it is desirable to use cells obtained by the differentiation of pluripotent stem cells (particularly iPS cells (especially human iPS cells)) into fibroblasts. The differentiation of pluripotent stem cells into stromal cells can be induced according to a known method. When the pluripotent stem cells are human iPS cells, for example, the differentiation of iPS cells can be induced into fibroblasts by the method described in PLoS ONE 8(10):e77673, 2013. Thus, the use of stromal cells derived from pluripotent stem cells makes it possible to provide a method for producing T cells with higher safety in production than when using primary cells or cell lines. Due to the use of stromal cells derived from pluripotent stem cells, there is little difference between lots of feeder cells used, and T cells can be produced stably. Furthermore, as shown in the Examples below, the use of stromal cells derived from pluripotent stem cells makes it possible to produce T cells with improved anticancer activity and cellular kinetics.

The Notch ligands are not particularly limited, and includes the canonical Notch ligand and non-canonical Notch ligand disclosed in WO2017/075389. Examples of the canonical Notch ligand include DLL4 (Delta-like ligand 4), DLL1 (Delta-like ligand 1), JAG1 (Jagged 1), JAG2 (Jagged 2), and the like. These can be used singly or in combination of two or more. Examples of the non-canonical Notch ligand include Contactin-1, NOV/CCN3, Contactin-6, Periostin/OSF-2, DLK2/EGFL9, Pref-1/DLK1/FA1, DNER, Thrombospondin-2, MAGP-1/MFAP2, Thrombospondin-3, MAGP-2/MFAP5, Thrombospondin-4, and Netrin-1. Human DLL4 is preferably used as the Notch ligand.

The means for introducing the nucleic acid for expressing Notch ligand(s) into stromal cells or pluripotent stem cells is not particularly limited, and various known or general means can be used. Typically, the nucleic acid for expressing Notch ligand(s) is introduced into stromal cells using an expression vector, and is expressed. The expression vector may be linear or cyclic, and may be a non-viral vector such as a plasmid, a viral vector, or a transposon vector.

The means for introducing the expression vector into stromal cells can be made appropriate according to the embodiment. For example, the expression vector can be introduced into stromal cells by a known method, such as a virus infection method, a calcium phosphate method, a lipofection method, a microinjection method, or an electroporation method. The expression vector can be prepared in a form suitable for use in each method by known means, or using commercially available kits as appropriate (according to the instructions thereof).

The expression vector can be introduced into stromal cells by a virus infection method. Examples of viral vectors include retroviral vectors, lentiviral vectors, adenoviral vectors, and adeno-associated viral vectors. When using these viral vectors, a vector containing the nucleic acid for expressing Notch ligand(s) and a packaging vector (plasmid) of each virus may be transfected into host cells using a corresponding commercially available kit to produce a recombinant virus, and then stromal cells may be infected with the obtained recombinant virus.

In addition to the nucleic acid for expressing Notch ligand(s), the expression vector may contain sequences, such as nuclear localization signal (NLS) and multi-cloning site (MCS), if necessary. The expression vector may further contain a nucleic acid (base sequence) encoding "functional genes," such as reporter genes (e.g., genes encoding various color fluorescent proteins), drug selection genes (e.g., kanamycin resistance gene, ampicillin resistance gene, and puromycin resistance gene), and suicide genes (e.g., genes encoding diphtheria A toxin, herpes simplex virus thymidine kinase (HSV-TK), carboxypeptidase G2 (CPG2), carboxylesterase (CA), cytosine deaminase (CD), cytochrome P450 (cyt-450), deoxycytidine kinase (dCK), nitroreductase (NR), purine nucleoside phosphorylase (PNP), thymidine phosphorylase (TP), varicella zoster virus thymidine kinase (VZV-TK), xanthine-guanine phosphoribosyltransferase (XGPRT), inducible caspase 9, etc.).

The cells that can differentiate into T cells and the stromal cells may be derived from humans or mammals other than humans (non-human mammals), and preferably human-derived cells. It is desirable to use human-derived cells from a zeno-free perspective. Examples of non-human mammals include mice, rats, hamsters, guinea pigs, rabbits, dogs, cats, pigs, cows, horses, sheep, and monkeys.

The three-dimensional cell aggregate(s) can be formed, for example, by centrifuging cells that can differentiate into T cells or cells differentiated therefrom, and stromal cells expressing Notch ligand(s). The ratio of the stromal cells to the cells that can differentiate into T cells is, for example, 100:1 to 1:100, 20:1 to 1:20, or 10:1 to 1:10, preferably 4:1 to 1:4, and more preferably 1:1.

The medium for culturing the three-dimensional cell aggregate(s) is not particularly limited, and examples include a serum-free medium, and particularly a serum-free medium containing insulin (further biotin, transferrin, and albumin).

Examples of the basal medium for culturing the three-dimensional cell aggregate(s) include AIMV, X-VIVO-15, NeuroBasal, EGM2, TeSR, BME, BGJb, CMRL1066, Glasgow's MEM, Improved MEM Zinc Option, IMDM, 199 medium, Eagle's MEM, αMEM, DMEM, Ham, RPMI-1640, Fischer's medium, and the like. These media may be used singly or as a mixture of two or more. In addition, for example, the medium components disclosed in WO2017/075389 may be suitably added.

As the culture conditions for culturing the three-dimensional cell aggregate(s), for example, the culture temperature is about 20 to 40°C, the CO₂ concentration is about 2 to 10%, the oxygen concentration is about 1 to 20%, and the culture period is, for example, about 1 to 12 weeks, preferably 3 to 10 weeks, and more preferably 6 to 9 weeks. The cell density at the start of culture can be, for example, about 1.0×10⁴ to 1.0×10¹⁰ cells/mL.

The medium, culture conditions, and the like used in the production method of the present invention can be those suitable for the type of cell to be cultured.

The basal medium used as such a medium is not particularly limited as long as it can be used to culture animal cells. Examples include those mentioned above.

The medium may contain serum or may be serum-free. The medium may further contain serum replacements (e.g., albumin, transferrin, Knockout Serum Replacement (KSR), fatty acid, insulin, collagen precursor, minor element, 2-mercaptoethanol, 3'-thiolglycerol, and ITS-supplement). Serum replacements can be used singly or in combination of two or more.

The medium may further contain one or more substances of lipids, amino acids (non-essential amino acids etc.), L-glutamine, vitamins, growth factors, cytokines, antibiotics, antioxidants, pyruvic acid, buffers, inorganic salts, etc. As the medium, it is desirable to use a chemically-defined medium that does not contain materials with unknown components, such as serum, because the difference in medium among lots can be reduced, and cells with stable quality can be prepared.

The pH of the medium is generally 7.0 to 7.8, and preferably 7.2 to 7.6. In order to prevent contamination before use, the medium is preferably sterilized by a method such as filtration, UV irradiation, heat sterilization, or radiation. The culture is carried out in the presence or absence of feeder cells. The culture conditions are not particularly limited, and conditions generally used for cell culture can be used. In the culture, passage may be performed as many times as necessary to obtain the desired amount of cells, or addition and replacement of medium may be performed. The culture container is not particularly limited, and can be suitably selected from plates, dishes, petri dishes, flasks, bags, bottles, tanks (culture tanks), bioreactors, and the like.

The production method of the present invention may further comprise separating the obtained T cells to concentrate the T cells. The T cells can be separated by a known method, such as a method using flow cytometry, or a magnetic cell separation method.

The T cells obtained by the production method of the present invention are CD3⁺ cells, and examples include helper T cells, which are CD4⁺ T cells, cytotoxic T cells, which are CD8⁺ T cells, regulatory T cells, Tfh cells (CXCR5⁺ cells), γδ T cells, NKT cells (CD56⁺ cells), naive T cells (CD45RA⁺CCR7⁺ cells), central memory T cells (CD45RA⁻CCR7⁺ cells), effector memory T cells (CD45RA⁻CCR7⁻ cells), terminal effector T cells (CD45RA⁺CCR7⁻ cells), and the like. Preferred among these are CD4⁺ T cells, CD8⁺ T cells, γδ T cells, and regulatory T cells. The T cells may be of one type only, or a mixture of two or more types.

In one aspect of the present invention, the production method of the present invention makes it possible to produce a cell population derived from pluripotent stem cells (preferably iPS cells (more preferably human iPS cells)), in which the content ratio of CD4⁺ T cells to CD8⁺ T cells is high in αβT cells contained in the cell population. The production method of the present invention is characterized in that it is possible to produce a cell population having a high content ratio of CD4⁺ T cells to CD8⁺ T cells in αβT cells, even without including a step of separating T cells in the process of producing T cells from cells that can differentiate into T cells. When the method does not include a step of separating T cells, the cell population can be produced more efficiently than when the method includes the step. The ratio (cell number) of CD4⁺ T cells to CD8⁺ T cells in αβT cells contained in the cell population containing CD4⁺ T cells and CD8⁺ T cells is, for example, 0.1 or more, 0.2 or more, 0.3 or more, 0.4 or more, 0.5 or more, 0.6 or more, 0.7 or more, 0.8 or more, 0.9 or more, 1.0 or more, 1.1 or more, 1.2 or more, 1.3 or more, 1.4 or more, 1.5 or more, 1.6 or more, 1.7 or more, 1.8 or more, 1.9 or more, 2.0 or more, 2.1 or more, 2.2 or more, 2.3 or more, 2.4 or more, 2.5 or more, 2.6 or more, 2.7 or more, 2.8 or more, 2.9 or more, 3.0 or more, 3.1 or more, 3.2 or more, 3.3 or more, 3.4 or more, 3.5 or more, 3.6 or more, 3.7 or more, 3.8 or more, 3.9 or more, or 4.0 or more. The ratio is preferably 0.6 or more, more preferably 1.0 or more, and even more preferably 2.0 or more. The upper limit of this ratio is, for example, 10, 9.0, 8.0, 7.0, 6.0, or 5.0.

The T cells obtained by the production method of the present invention may be T cells into which a foreign gene has been introduced.

The "foreign gene" is a gene to be introduced from the outside in order to express the desired protein in the T cells, and can be suitably selected depending on the use of the T cells.

One or more genes may be introduced as the foreign gene into the T cells obtained by the production method of the present invention, and the foreign gene may be, for example, a nucleic acid encoding an exogenous T cell receptor (TCR) or a chimeric antigen receptor (CAR). The foreign gene can be, for example, a gene for expressing a CAR, and can further contain a gene for expressing a cytokine and/or a chemokine (e.g., a fusion protein in which IL-15 and IL-15Rα are linked (IL-15Rα/IL-15)). As with general or known CARs, the CARs expressed by the T cells are basically configured such that peptides at sites of (i) an antigen recognition site that recognizes cell surface antigens of cancer cells (e.g., single-chain antibody), (ii) a transmembrane region, and (iii) a signal transduction region that induces the activation of T cells, are linked via a spacer, as needed. The TCR may be a dimer of α- and β-chains or γ- and δ-chains. The exogenous TCR means that it is exogenous to T cells into which a nucleic acid encoding the exogenous TCR is to be introduced. The amino acid sequence of the exogenous TCR may be identical to or different from that of the endogenous TCR of the T cells. When the foreign gene to be introduced is a nucleic acid encoding an exogenous T-cell receptor, an example of the nucleic acid is a nucleic acid encoding Vg9Vd2TCR.

Examples of the means for introducing the foreign gene into the cells include the methods mentioned above. The cells into which the foreign gene is introduced are not particularly limited, and may be at any stage of differentiation. Examples include pluripotent stem cells (particularly iPS cells), cells that can differentiate into T cells, T cells, and the like.

Other examples of one or more foreign genes introduced into the T cells obtained by the production method of the present invention include an expression construct comprising (a) CNS1 (conserved non-coding sequence 1), CNS2 (conserved non-coding sequence 2), and CNS3 (conserved non-coding sequence 3) of Foxp3 gene, (b) a promoter, and (c) a nucleic acid encoding FOXP3 (which may be simply referred to as "expression construct" or "CNS-Foxp3" in the present specification). Regulatory T cells can be produced with high efficiency by introducing such an expression construct into pluripotent stem cells, cells that can differentiate into regulatory T cells, or the like. The expression construct is not particularly limited as long as it can express FOXP3. The expression construct preferably contains a terminator, a polyadenylation signal, Foxp3 3'UTR, etc., in addition to (a), (b), and (c) mentioned above, and is more preferably one that functions in cells into which these are introduced.

The base sequences of human Foxp3 gene are registered as RefSeq Accession Nos. NM_001114377 (SEQ ID No. 1) and NM_014009 (SEQ ID No. 2), and the amino acid sequences thereof are also registered as RefSeq Accession Nos. NP_001107849 (SEQ ID No. 3) and NP_054728 (SEQ ID No. 4). These RefSeq IDs are registered on the NCBI website. In the present invention, the above gene also includes degenerate products and variants of genes other than those having the base sequences registered in the database mentioned above. Desirable variants are those encoding proteins having a biological activity equivalent to that of the protein consisting of the above amino acid sequences. Examples of variants include FOXP3 variants having an amino acid sequence that is 80% or more, 85% or more, 90% or more, 910 or more, 92% or more, 93% or more, 94% or more, 950 or more, 960 or more, 970 or more, 980 or more, or 99% or more, identical to the natural amino acid sequence. In the present specification, FOXP3 refers to a protein, and Foxp3 refers to a gene; however, FOXP3 and Foxp3 refer to a gene and protein, respectively, when such an interpretation is appropriate.

The nucleic acid encoding FOXP3 is not particularly limited as long as it is a nucleic acid encoding FOXP3 protein, and preferably cDNA of FOXP3.

CNS1, CNS2, and CNS3 used in the present invention are all derived from Foxp3 gene. CNS1, CNS2, and CNS3 are Foxp3 enhancer elements. The promoter used in the present invention is not particularly limited, and examples include CAG promoter, ubiquitin gene promoter, Foxp3 gene promoter, EF1α promoter, SRα promoter, SV40 promoter, LTR promoter, cytomegalovirus (CMV) promoter, Rous sarcoma virus (RSV) promoter, Moloney murine leukemia virus (MoMuLV) LTR, herpes simplex virus thymidine kinase (HSV-TK) promoter, and the like; and preferably Foxp3 gene promoter. Preferred examples of the base sequences of CNS1, CNS2, CNS3, and promoter of human Foxp3 gene include the base sequences represented by SEQ ID Nos. 5 to 8, respectively. The promoter, CNS1, CNS2, and CNS3 include variants even if they do not have the base sequences described above. Desirable variants are those having a biological activity equivalent to that of the promoter, CNS1, CNS2, and CNS3 consisting of the above base sequences. Examples of variants include those having a base sequence that is 80% or more, 85% or more, 90% or more, 910 or more, 92% or more, 93% or more, 94% or more, 950 or more, 960 or more, 970 or more, 980 or more, or 99% or more, identical to the natural base sequence.

The arrangement (order) of the promoter, CNS1, CNS2, CNS3, and nucleic acid encoding FOXP3 in the expression construct is not particularly limited. It is preferable that CNS1, CNS2, and CNS3 are located upstream of the promoter; and the order of CNS1, CNS2, CNS3, promoter, and nucleic acid encoding FOXP3 from the 5'-terminal side is more preferred.

It is desirable to use pluripotent stem cells (particularly iPS cells) as the cells into which the expression construct is introduced, induce differentiation of these cells into CD34⁺ cells (particularly hemogenic endothelial cells), and then subject the CD34⁺ cells (particularly hemogenic endothelial cells) to the production method of the present invention to differentiate them into regulatory T cells.

The T cells produced by the production method of the present invention are useful for the prevention and/or treatment of T cell-related diseases. When the T cells produced by the production method of the present invention are CD4⁺ T cells, CD8⁺ T cells, or γδ T cells, these cells are useful for, for example, but are not limited to, the treatment and prevention of tumors (blood tumors and solid tumors), infections, blood diseases, and the like. When the T cells produced by the production method of the present invention are regulatory T cells, the cells are useful for the treatment and prevention of animals (in particular, humans) with an abnormally enhanced immune response. For example, the regulatory T cells are useful for the treatment and prevention of immune dysregulation, polyendocrinopathy, enteropathy, X-linked (IPEX) syndrome, graft-versus-host disease (GVHD), rejection in organ transplantation, autoimmune diseases, inflammatory diseases, allergic diseases (hay fever, asthma, atopic dermatitis, eczema, food allergy, food hypersensitivity, urticaria, allergic rhinitis, allergic conjunctivitis, and drug allergy), and the like. The T cells produced by the production method of the present invention may be used for autologous transplantation or allogeneic transplantation. Further, the T cells may be used in combination with other drugs.

When performing such cell therapy, from the viewpoint that rejection does not occur, the subject from which cells are isolated for use in the production of T cells preferably has the same HLA type as a subject to which T cells are to be administered, and is more preferably the same as the subject to which T cells are to be administered.

The present invention can produce a medicine comprising T cells (hereinafter also referred to as "the medicine of the present invention"). The medicine of the present invention is preferably produced as a parenteral preparation by mixing an effective amount of T cells with a pharmaceutically acceptable carrier according to known means (e.g., the methods described in the Japanese Pharmacopoeia). The medicine of the present invention is preferably produced as a parenteral preparation, such as injection, suspension, or infusion. Examples of parenteral administration methods include intravenous, intraarterial, intramuscular, intraperitoneal, or subcutaneous administration. Examples of the pharmaceutically acceptable carrier include solvents, bases, diluents, excipients, soothing agents, buffers, preservatives, stabilizers, suspensions, isotonic agents, surfactants, solubilizing agents, and the like.

The dose of the medicine of the present invention can be suitably determined depending on various conditions, such as patient's body weight, age, sex, and symptoms. In general, the medicine of the present invention is administered so that the number of cells per administration for a subject with a body weight of 60 kg is generally 1×10⁶ to 1×10¹⁰ cells, preferably 1×10⁷ to 1×10⁹ cells, and more preferably 5×10⁷ to 5×10⁸ cells. The medicine of the present invention may be administered once or several times. The medicine of the present invention can have a known form suitable for parenteral administration, such as injection or infusion. Further, the medicine of the present invention may contain physiological saline, phosphate buffered saline (PBS), medium, and the like in order to stably maintain the cells. The medium may be, for example, but is not limited to, RPMI, AIM-V, X-VIVO10, or other media. In addition, pharmaceutically acceptable carriers (e.g., human serum albumin), preservatives, and the like may be added to the medicine for the purpose of stabilization. The medicine of the present invention is applied to mammals, including humans.

As used in the present specification and claims, unless otherwise required by context, singular terms shall include pluralities and plural terms shall include the singular. Thus, singular articles (e.g., "a," "an," "the," and the like in the case of English) should also be understood as encompassing the concepts thereof in the plural form unless specifically noted otherwise.

### Examples

Examples are provided below in order to explain the present invention in more detail. However, the present invention is not limited to these Examples.

### 1) Preparation of iPS Cell-Derived Fibroblasts/DLL4 Cells

For use as feeder cells when inducing differentiation of T cells from iPS cells, fibroblasts were differentiated from iPS cells (Ff-I01s04 strain: derived from healthy peripheral blood mononuclear cells) provided by the Center for iPS Cell Research and Application, Kyoto University. Subsequently, lentivirus was used to produce cells forcibly expressing DLL4, which is a Notch ligand, under the control of the EFlalpha promoter.

Specifically, the Ff-I01s04 strain was seeded in a medium supplemented with AK03N (Ajinomoto Co., Inc.), Y-27632 (FUJIFILM Wako Pure Chemical Corporation), and CHIR990211 (Tocris) in a 6-well plate (Corning), which had been subjected to ultra-low adhesion treatment, at 1×10⁶ cells/well (Day 0). The next day, the cells were seeded in a fibroblast medium (alpha-MEM (Invitrogen) supplemented with 20% FBS (Corning), 10 ug/ml human insulin, 5.5 ug/ml human transferrin, 5 ng/ml sodium selenite (ITS, Gibco), 2 mM L-glutamine, 100 U/ml penicillin, 100 ug/ml streptomycin (PSG, Sigma-Aldrich), and 50 ug/ml ascorbic acid 2-phosphate (Sigma-Aldrich)) in a 10 cm dish (Corning) coated with 0.1% gelatin (Nacalai Tesque, Inc.) (Day 1). The medium was changed twice a week, and the culture was continued for 7 days to obtain a cell population containing fibroblasts (Day 8).

The obtained iPS cell-derived fibroblasts were seeded at 1×10⁵ cells/well in a gelatin-coated 24-well plate, and the next day, a solution of lentivirus carrying human DLL4 gene introduced downstream of the EFlalpha promoter and protamine at a final concentration of 10 ug/mL were added to prepare iPS cell-derived Fibroblast/DLL4 cells (iFibro/DLL4).

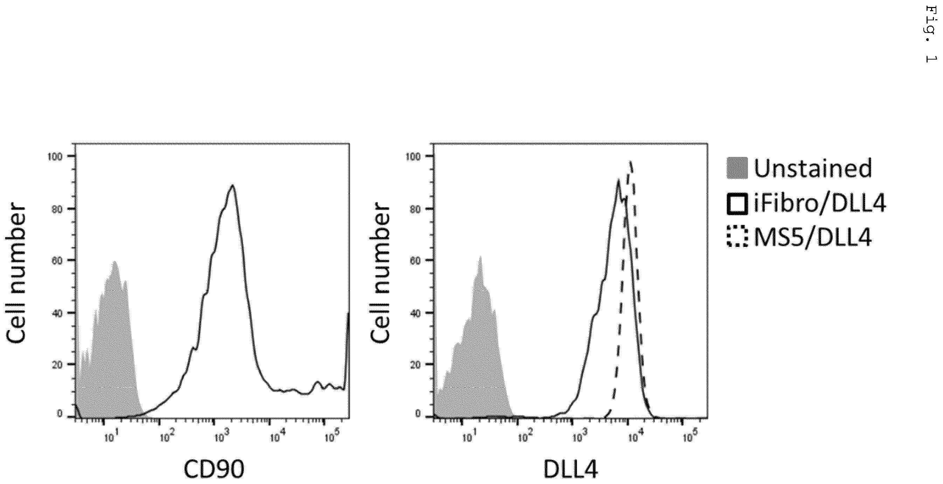

The prepared cells were stained with anti-DLL4 antibody-APC (BioLegend) or anti-CD90 antibody-APC (BioLegend), which is a fibroblast marker, and analyzed by flow cytometry. As a result, expression of DLL4 and the fibroblast marker was observed, as shown in Fig. 1.

### 2) Preparation of Vg9Vd2TCR-Introduced iPS Cells (QHJI01s04/G9D2 Cells)

Vg9Vd2TCR gene was introduced into iPS cells (QHJI01s04 strain: derived from healthy peripheral blood mononuclear cells) provided by the Center for iPS Cell Research and Application, Kyoto University, according to the method described in WO2020/013315 to prepare Vg9Vd2TCR-introduced iPS cells.

Specifically, the iPS cells were seeded in a 24-well plate at 1×10⁴ cells/well, and the next day, a solution of lentivirus carrying Vg9Vd2 TCR gene incorporated downstream of the human ubiquitin promoter and protamine at a final concentration of 10 ug/mL were added to prepare QHJI01s04/G9D2 cells.

### 3) Induction of Differentiation from iPS Cells into HEC

The cell population containing hemogenic endothelial cells used was a floating cell population differentiated from iPS cells (Ff-I01s04 strain: derived from healthy peripheral blood mononuclear cells) provided by the Center for iPS Cell Research and Application, Kyoto University, by known methods (e.g., the methods disclosed in Cell Reports 2 (2012) 1722-1735 and WO2017/221975).

Specifically, the Ff-I01s04 strain was seeded in a 6-well plate (Corning), which had been subjected to ultra-low adhesion treatment, at 1×10⁶ cells/well (Day 0). 50 ng/ml BMP4 (R&D systems), 50 ng/ml bFGF (FUJIFILM Wako Pure Chemical Corporation), 50 ng/ml VEGF (R&D systems), and 2 µM SB431542 (FUJIFILM Wako Pure Chemical Corporation) were added to EB medium (StemPro34 (Gibco) supplemented with 10 ug/ml human insulin, 5.5 ug/ml human transferrin, 5 ng/ml sodium selenite (ITS, Gibco), 2 mM L-glutamine (Sigma-Aldrich), 45 mM α-monothioglycerol (Nacalai Tesque, Inc.), and 50 ug/ml ascorbic acid 2-phosphate (Sigma-Aldrich)), and the cells were cultured under low oxygen conditions (50 O₂) for 4 days (Day 4). Subsequently, the cells were further cultured for 4 days in a medium supplemented with 50 ng/ml bFGF, 50 ng/ml VEGF, and 50 ng/ml SCF (R&D systems) (Day 8), thereby obtaining a cell population containing HEC. The floating cell population containing HEC was stained using the antibody set in Table 1 below.

**Table 1**

| Antibody | Vendor | Fluorescent label |
|---|---|---|
| Anti-CD34 antibody | Abeam | PE/Cy7 |
| Anti-CD43 antibody | BD | BV510 |
| Anti-CD73 antibody | BD | APC |
| Anti-CD184 antibody | BD | BV421 |
| Anti-CD235a antibody | BioLegend | FITC |
| Anti-CD14 antibody | BioLegend | APC/ef780 |

It was confirmed by a flow cytometry method that the obtained cell population contained about 15% of HEC (CD34⁺/CD43⁻/CD73⁻/CD184⁻ cells). Further, a cell population containing HEC was obtained from QHJI01s04/G9D2 cells in the same manner as described above, except for using the QHJI01s04/G9D2 cells produced in 2) above in place of the Ff-I01s04 strain.

### 4) Induction of T-cell Differentiation

iFibro/DLL4 obtained in 1) above and the cell population containing HEC obtained in 3) above were allowed to differentiate into T cells with reference to the description in WO2017/075389 etc.

Specifically, iFibro/DLL4 or mouse stromal cell strain MS5 in which DLL4 was forcibly expressed (MS5/DLL4) was used as a support, and each co-cultured with the iPS cell-derived HEC produced in 3) above at a cell ratio of 1:1 or 4:1. The medium used was RPMI-1640 (FUJIFILM Wako Pure Chemical Corporation) containing, at a final concentration, 2x B27 supplement (Invitrogen), 1x PSG (Sigma-Aldrich), 1x Glutamax (Invitrogen), 5 ng/mL IL-7 (PeproTech), 5 ng/mL FlT3L (PeproTech), and 50 ug/mL ascorbic acid (Sigma-Aldrich). The cells co-cultured on 30 mm Millicell (hydrophilic PTFE, pore size: 0.4 µm, height: 5 mm, Merck Millipore) were allowed to stand in a 6-well plate (TPP), and cultured for 9 weeks while changing the medium once every three or four days.

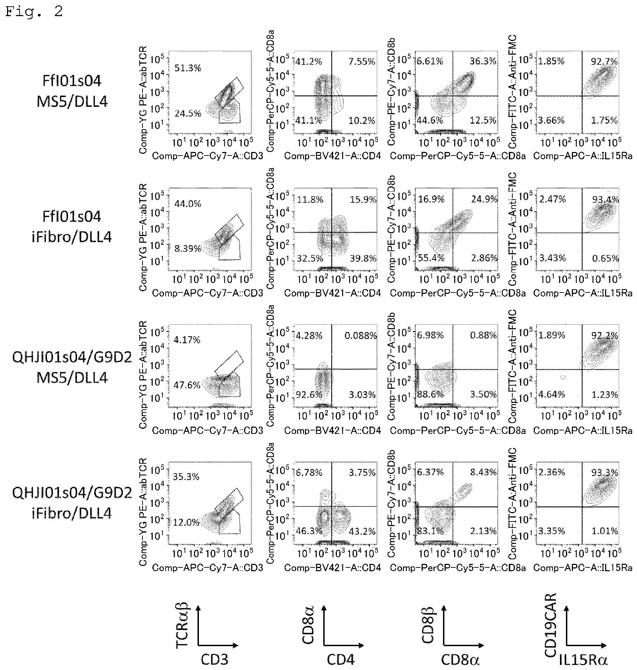

After the culture, the obtained cells were collected and expanded according to the method described in WO2020/032179, and then anti-CD19-CAR (SEQ ID No: 9) gene and IL-15Rα/IL-15 (SEQ ID No: 10) gene were introduced. As a result, iPS cell-derived CD19CAR T cells were obtained. Intracellular staining was performed using the antibody set in Table 2 below, and the expression of T-cell surface molecules, as well as anti-CD19 CAR and IL-15Rα, was confirmed by flow cytometry as shown in Fig. 2.

**Table 2**

| Antibody | Vendor | Fluorescent dye |
|---|---|---|
| Anti-CD3∑ antibody | BioLegend | APC/Cy7 |
| Anti-CD4 antibody | BioLegend | BV421 |
| Anti-CD8α antibody | BioLegend | PerCP/Cy5.5 |
| Anti-CD8β antibody | eBioscience | PE/Cy7 |
| Anti-TCRαβ antibody | eBioscience | PE |
| Anti-IL15Rα antibody | BioLegend | APC |
| Anti-CD19CAR antibody | Aerobic | FITC |

### 5) In Vivo Efficacy and Cellular Kinetics (CK)

The drug efficacy and cellular kinetics of the iPS cell-derived CD19CAR T cells prepared in 4) above were examined in an immunodeficient mouse cancer model.

Specifically, Nalm6 (human B-cell leukemia cell-derived cell line) transfected with luciferase at 5×10⁵ cells/200 µL per mouse was intravenously administered to 8-week-old NOD.Cg-Prkdc^{scid}Il2rg^{tm1Wjl}/SzJ (NSG) mice (Charles River Japan, Inc.), and 4 days later, the CD19CAR T cells prepared in 4) above were intravenously administered in the group composition shown in Table 3.

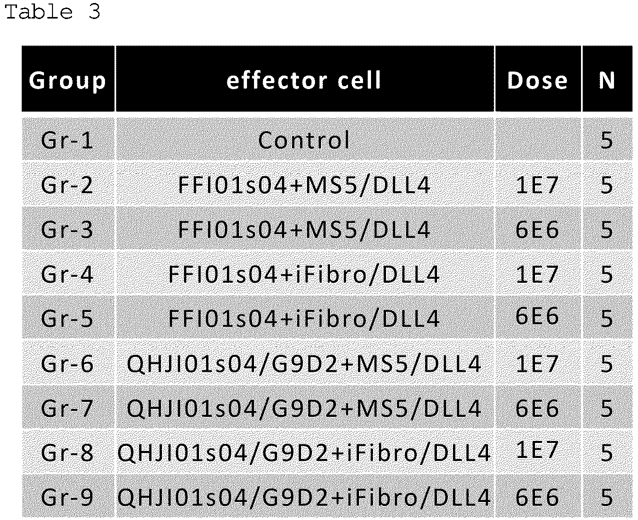

Starting one week after administration of the CD19CAR T cells, 3 mg of VivoGlo luciferin (Promega) was administered intraperitoneally per mouse, and 10 minutes later, the remaining cancer cells were visualized using IVIS Lumina II (Perkin Elmer). At the same time, body weight was measured and blood was collected using heparinized tubes. The collected blood was stained with the antibody set in Table 4, then hemolyzed and fixed using FACS lysing solution (BD), and analyzed using FACS LSR Fortessa (BD). CK was calculated by regarding CD45⁺ cells as CD19CAR T cells.

**Table 4**

| Antibody | Vendor | Fluorescent dye |
|---|---|---|
| Anti-CD45 antibody | BioLegend | BV510 |
| Anti-CD4 antibody | BioLegend | BV421 |
| Anti-CD19 antibody | BioLegend | APC/Cy7 |
| Anti-CD8β antibody | eBioscience | PE/Cy7 |
| Anti-CD3 antibody | BioLegend | APC |
| Anti-TCRαβ antibody | eBioscience | PE |

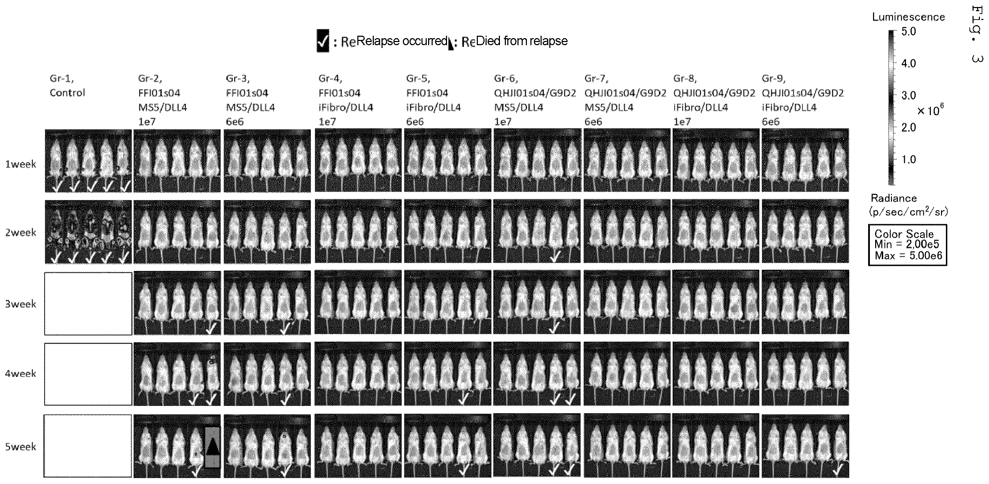

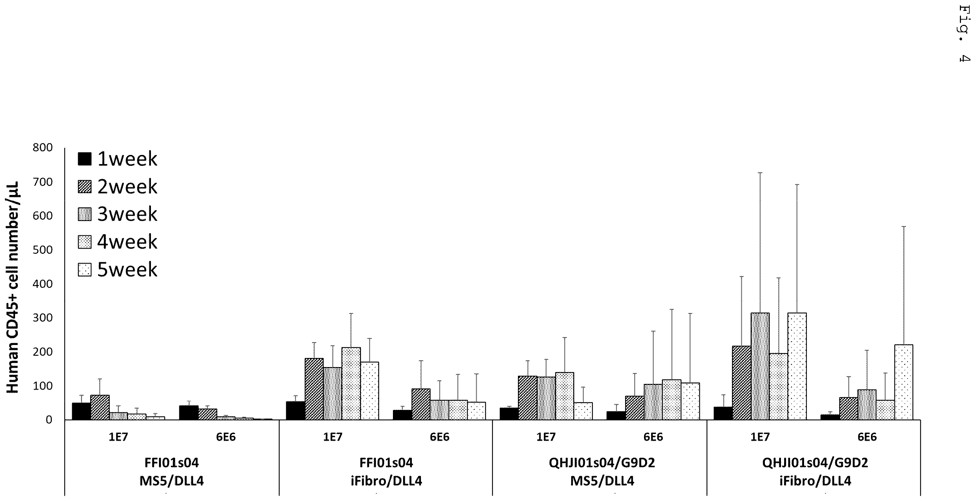

As a result, as shown in Fig. 3, the number of cancer cells was significantly reduced in all CD19CAR T-cell administration groups compared to the control group, demonstrating strong drug efficacy. Furthermore, in a comparison between the CD19CAR T cells, cells prepared using iFibro/DLL4 as a support showed stronger drug efficacy than cells prepared using MS5/DLL4 as a support. Further, as shown in Fig. 4, the number of CD19CAR T cells remaining in the mouse body tended to be positively correlated with drug efficacy.

### 6) Proportion of CD4⁺ T cells and CD8⁺ T cells in Differentiated T-Cell Population

In the iPS cell-derived T-cell population prepared in 4) above (cell population before introduction of anti-CD19-CAR gene and IL-15Rα/IL-15 gene), the proportion (%) of CD4⁺ T cells and CD8⁺ T cells in αβ T cells, and the ratio of CD4⁺ T cells to CD8⁺ T cells were calculated. As a result, as shown in Table 5, it was found that the T-cell population prepared using iFibro/DLL4 as a support had a higher proportion of CD4⁺ T cells than the T-cell population prepared using MS5/DLL4 as a support (the proportions of CD4⁺ T cells and CD8⁺ T cells in Table 5 show average values).

**Table 5**

| | CD4⁺/ CD3⁺TCRab⁺ | CD8⁺/ CD3⁺TCRab⁺ | CD4/CD8 ratio CD4/CD8 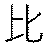 |
|---|---|---|---|
| Ffl01s04+MS5/DLL4 (N=8) | 31.3 | 53.8 | 0.58 |
| Ffl01s04+iFibro/DLL4 (N=9) | 42.1 | 21.7 | 1.94 |
| QHJI01s04/G9D2+MS5/DLL4 (N=3) | 7.1 | 50.5 | 0.14 |
| QHJI01s04/G9D2+iFibro/DLL4 (N=4) | 63.8 | 14.6 | 4.36 |

### 7) Examination of Differentiation from iPS Cells into Treg and Protein Expression Levels

Differentiation from iPS cells into Treg was specifically as follows. A plasmid sequence was designed and synthesized in which a DNA sequence in which mStrawberry protein sequence registered in GenBank (MK012431) was changed to dTomato sequence was incorporated into a transfer plasmid for producing a third-generation lentiviral vector. This plasmid was transfected into cells of the HEK293 line together with a packaging plasmid and an envelope plasmid for producing a lentiviral vector, and the supernatant containing the produced lentiviral vector was collected and then concentrated by high-speed centrifugation to obtain a lentiviral vector to be introduced into iPS cells. Next, the Ff-I01s04 strain was seeded in a 24-well plate at 1×10⁴ cells/well, protamine was added to a final concentration of 10 µg/mL, and then a solution of lentivirus carrying CNS-Foxp3 incorporated was directly added to produce virus-infected iPS cells.

The virally infected Ff-I01s04 strain was seeded in a 6-well plate (Corning), which had been subjected to ultra-low adhesion treatment, at 1×10⁶ cells/well (Day 0). 50 ng/ml BMP4 (R&D systems), 50 ng/ml bFGF (FUJIFILM Wako Pure Chemical Corporation), 50 ng/ml VEGF (R&D systems), and 2 µM SB431542 (FUJIFILM Wako Pure Chemical Corporation) were added to EB medium (StemPro34 (Gibco) supplemented with 10 ug/ml human insulin, 5.5 ug/ml human transferrin, 5 ng/ml sodium selenite (ITS, Gibco), 2 mM L-glutamine (Sigma-Aldrich), 45 mM α-monothioglycerol (Nacalai Tesque, Inc.), and 50 ug/ml ascorbic acid 2-phosphate (Sigma-Aldrich)), and the cells were cultured under low oxygen conditions (50 O₂) for 4 days. Subsequently, the cells were further cultured for 4 days in a medium supplemented with 50 ng/ml bFGF, 50 ng/ml VEGF, and 50 ng/ml SCF (R&D systems), thereby obtaining a cell population containing HEC. Further, iFibro/DLL4 obtained in 1) above was used as a support and co-cultured with CNS-Foxp3-introduced human iPS cell-derived HEC at a cell ratio of 1:1. The medium used was RPMI-1640 (FUJIFILM Wako Pure Chemical Corporation) containing, at a final concentration, 2x B27 supplement (Invitrogen), 1x PSG (Sigma-Aldrich), 1x Glutamax (Invitrogen), 5 ng/mL IL-7 (PeproTech), 5 ng/mL FlT3L (PeproTech), and 50 ug/mL ascorbic acid (Sigma-Aldrich). The cells co-cultured on 30 mm Millicell (hydrophilic PTFE, pore size: 0.4 µm, height: 5 mm, Merck Millipore) were allowed to stand in a 6-well plate (TPP), and cultured for 9 weeks while changing the medium once every three or four days.

The cell population containing the CNS-Foxp3-introduced human iPS cell-derived Treg obtained above was seeded at 2.5×10⁵ cells/well in an anti-CD3 antibody (eBioscience)-binding 48-well cell culture plate, cultured for 3 days in a CO₂ incubator under the conditions of 37°C and 5.0% CO₂, and then reseeded in a 24-well G-Rex cell culture plate, and culture was continued. The medium used was α-MEM (Invitrogen) containing, at a final concentration, FBS (15%, Corning), an L-Glutamine-Penicillin-Streptomycin solution (1/100, Invitrogen, Sigma-Aldrich), Insulin-Transferrin-Selenium Supplement (1/100, Invitrogen), ascorbic acid 2-phosphate (50 µg/mL, Sigma-Aldrich), IL-2 (10 ng/mL, PeproTech), anti-CD30 antibody (1.5 µg/mL, R&D systems), rapamycin (10 nM, LKT Laboratories), and anti-TNFR2 antibody (3 µg/mL, Hycult Biotech). For the first three days, anti-CD28 antibody (1.5 µg/mL, BioLegend) was further added to the above formulation. The cells thus obtained were isolated according to the recommended protocol for CD4 MicroBeads (Miltenyi Biotec) to obtain CD4-positive cells. The cells were expanded once in the same manner (day 0), and then cultured again for 18 days (day 18) in the same medium further supplemented with IL-3 (60 ng/mL, BioLegend), IL-4 (30 ng/mL, BioLegend), IL-33 (30 ng/mL, BioLegend), and TGF-β1 (5 ng/mL, BioLegend). Each cell population was stained with the following antibody set (Panel 1: Zombie NIR, BV510 CD3, BV421 CD4, PE/Cy7 CD8b, APC CD25, PE GZMB, AF488 FOXP3, PerCP/Cy5.5 CTLA4; Panel 2: Zombie NIR, BV510 CD3, BV421 CD4, PE/Cy7 CD8b, AF488 FOXP3, PE Helios).

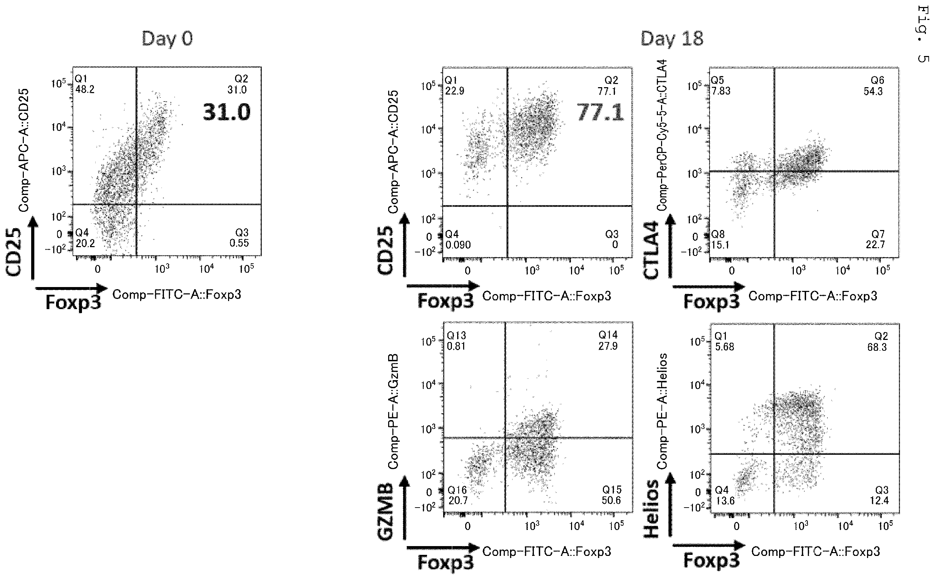

As a result, as shown in Fig. 5, 31.0% of the cells were differentiated into Treg in the first expansion culture (Day 0), and the Treg population was further enriched to 77.1% through the second expansion culture (Day 18).

The present application is based on Japanese Patent Application No. 2022-016271 filed on February 4, 2022 in Japan, the entire contents of which are incorporated herein.

Sequence Listing

## Claims

1. A method for producing T cells, the method comprising:
(1) culturing three-dimensional cell aggregate(s) comprising cells that can differentiate into T cells, and stromal cells that express Notch ligand(s) derived from pluripotent stem cells.

2. The method according to claim 1, wherein the cells that can differentiate into T cells are CD34⁺ cells or mesodermal progenitor cells.

3. The method according to claim 1, wherein the cells that can differentiate into T cells are hematopoietic stem cells, hemogenic endothelial cells, progenitor T cells, or mesodermal progenitor cells.

4. The method according to claim 1, wherein the cells that can differentiate into T cells are hemogenic endothelial cells.

5. The method according to claim 1, wherein the cells that can differentiate into T cells are cells derived from pluripotent stem cells.

6. The method according to claim 1, wherein the cells that can differentiate into T cells are cells derived from iPS cells.

7. The method according to claim 1, wherein the stromal cells are fibroblasts.

8. The method according to claim 1, wherein the pluripotent cells are iPS cells.

9. The method according to claim 1, wherein the Notch ligand(s) is at least one member selected from the group consisting of DLL4, DLL1, JAG1, and JAG2.

10. The method according to claim 1, further comprising, before step (1):
(2) inducing differentiation of pluripotent stem cells into stromal cells.

11. The method according to claim 6, further comprising, before step (1):
(3) inducing differentiation of iPS cells into cells that can differentiate into T cells.

12. The method according to claim 1, further comprising, before step (1):
(4) introducing a nucleic acid encoding an exogenous T-cell receptor and/or a chimeric antigen receptor into cells that can differentiate into T cells.

13. The method according to claim 11, further comprising, before step (3):
(5) introducing a nucleic acid encoding an exogenous T-cell receptor and/or a chimeric antigen receptor into iPS cells.

14. The method according to claim 1, which is a method for producing T cells comprising at least one selected from the group consisting of CD4⁺ T cells, CD8⁺ T cells, γδ T cells, and regulatory T cells.

15. T cells obtained by the method according to claim 1.

16. A medicine comprising the T cells according to claim 15.

17. A method for producing T cells expressing an exogenous T-cell receptor and/or a chimeric antigen receptor, comprising:
(6) introducing a nucleic acid encoding an exogenous T-cell receptor and/or a chimeric antigen receptor into T cells obtained by the method according to claim 1.

18. The medicine according to claim 16, which is a preventive and/or therapeutic agent for a T cell-related disease.

19. The medicine according to claim 18, wherein the T cell-related disease is a tumor, an infection, or a blood disease.

20. A method for preventing and/or treating a T cell-related disease, comprising administering the T cells according to claim 15 to a subject in need thereof.

21. The method according to claim 20, wherein the T cell-related disease is a tumor, an infection, or a blood disease.

22. The T cells according to claim 15 for use in the prevention and/or treatment of a T cell-related disease.

23. The T cells according to claim 22, wherein the T cell-related disease is a tumor, an infection, or a blood disease.

24. Use of the T cells according to claim 15 in the production of a medicine for use in the prevention and/or treatment of a T cell-related disease.

25. The use according to claim 24, wherein the T cell-related disease is a tumor, an infection, or a blood disease.
